# EUROPÄISCHE PATENTANMELDUNG

(11) **EP 1 867 986 A1**
(43) Veröffentlichungstag der Anmeldung: **19.12.2007**
(21) Anmeldenummer: 06012194.4
(22) Anmeldetag: 13.06.2006
(51) Int. Cl.: G01N 33/49, G01N 33/86, G01N 21/03, G01N 35/02, B01L 3/00, G01N 21/07

(54) **Vorrichtung zum Untersuchen von Körperflüssigkeiten**

(71) Anmelder: Behnk, Holger, D-22417 Hamburg (DE)
(72) Erfinder: Behnk, Holger, D-22417 Hamburg (DE)
(74) Vertreter: Glawe, Delfs, Moll

(57) **Zusammenfassung**

Die Vorrichtung zum Untersuchen von Körperflüssigkeiten, die mehrere Küvetten (8) aufweist, in die Körperflüssigkeit und ein Reagenz einbringbar ist, zeichnet sich dadurch aus, dass die Vorrichtung nach oben offene Kammern (31) aufweist, deren Anzahl mindestens gleichgroß ist wie diejenige der Küvetten (8).

## Beschreibung

Die Erfindung betrifft eine Vorrichtung zum Untersuchen von Körperflüssigkeiten, die mehrere Küvetten aufweist, in die Körperflüssigkeit und ein Reagenz einbringbar ist.

Körperflüssigkeiten, die untersucht werden sollen, werden dem Labor in Glasröhrchen geliefert, die mit einem Stopfen verschlossen sind. Die Körperflüssigkeit möchte man dabei aus Gründen der Hygiene und um Übertragung von Krankheiten und Kontaminierung der Körperflüssigkeit zu vermeiden, entnehmen, ohne den Stopfen zu entfernen. Zu diesem Zweck ist es bekannt, den Stopfen mit einer zu einer Injektionsnadel ähnlichen Nadel zu durchstoßen und dann die gewünschte Flüssigkeitsmenge heraus zu saugen.

Das Problem dabei ist, dass der Stopfen das Glasröhrchen luftdicht verschließen muss. Es herrscht daher in Glasröhrchen ein Unterdruck oder ein Überdruck, dessen Größe nicht bekannt ist. Je nach Innendruck des Glasröhrchens wird weniger oder mehr Körperflüssigkeit entnommen, als dies gewünscht ist. Ganz offensichtlich werden dadurch die Messergebnisse verfälscht, da die Reaktionsgeschwindigkeit zwischen Körperflüssigkeit und Reagenz natürlich vom Mischungsverhältnis dieser beiden abhängt.

Es ist bekannt, die Nadel an ihrem Außenumfang mit sich in Längsrichtung erstreckenden Rillen zu versehen, so dass zwischen Nadel und Stopfen Luftkanäle verbleiben, wenn die Nadel in den Stopfen hineingestochen ist. Der entsprechende Druckausgleich wird aber trotzdem unter Umständen nicht erreicht, da das weiche Material des Stopfens die Luftkanäle zusetzen kann. Außerdem wird die Nadel wegen der Rillen einen größeren Durchmesser haben, so dass es schwieriger ist, sie durch den Stopfen zu stoßen. Es ist auch bekannt, statt der Rillen geschlossene Luftkanäle vorzusehen, was die Herstellung der Nadel sehr aufwändig macht. Auch eine solche Nadel wird selbstverständlich einen größeren Durchmesser haben müssen, was mit dem vorstehend genannten Nachteil verbunden ist. Außerdem wird der seitliche Druckausgleichkanal beim Herausziehen der Nadel verschlossen, so dass dann beim weiteren Herausziehen ein Unterdruck erzeugt wird, der einen Teil der aufgenommenen Flüssigkeit wieder aus der Nadel heraussaugt. Dieses Problem tritt auch bei einem weiteren vorbekannten System auf, bei dem zwei Nadeln verwendet werden, nämlich eine Nadel zum Heraussaugen der Flüssigkeit und eine weitere mit dem Kanal für den Druckausgleich. Auch hier sind wieder größere Kräfte aufzuwenden, um die Nadeln durch den Stopfen zu stoßen, als dies bei einer Nadel der Fall wäre.

Die Aufgabe der Erfindung besteht in der Schaffung einer Vorrichtung der eingangs genannten Art, mit der auch im Falle von mit Stopfen verschlossenen Glasröhrchen genau definierte Mengen der Körperflüssigkeit untersucht werden können. Es soll also die gleiche Genauigkeit erhalten werden wie im Falle von nicht durch Stopfen verschlossenen Probenröhrchen.

Die erfindungsgemäße Lösung besteht darin, dass die Vorrichtung nach oben offene Kammern aufweist, deren Anzahl mindestens gleichgroß ist wie diejenige der Küvetten.

Mit der erfindungsgemäßen Vorrichtung ist folgende Arbeitsweise möglich. Es wird zunächst mit einer Nadel, die keine Rillen oder Kanäle für Druckausgleich haben muss, eine größere Flüssigkeitsmenge entnommen, als für die Messung erforderlich ist. Diese Flüssigkeitsmenge wird dann in eine der Kammern eingegeben. Aus dieser Kammer kann dann die Flüssigkeit drucklos und damit genau dosierbar in die Küvette eingegeben werden. Es ist also eine genaue Dosierung und damit Messung möglich, ohne dass wesentlicher Aufwand bei der Herstellung der Vorrichtung getrieben werden muss. Es ist auch ohne weiteres möglich, die Kammern zwischen den Küvetten anzuordnen und zum Beispiel einstückig mit der Halterung der Küvetten zum Beispiel durch Spritzgiessen herzustellen. Es entstehen also keine großen zusätzlichen Kosten. Die Kammern müssen auch nicht separat steril gehalten werden oder separat entsorgt werden. Die Handhabung geschieht vielmehr zusammen mit den Küvetten.

Zweckmäßigerweise ist die Anzahl der offenen Kammern gleich der Anzahl der Küvetten. Die offenen Kammern sind dabei zweckmäßigerweise zwischen Küvetten angeordnet, da so nur wenig zusätzlicher Raum eingenommen wird. Als besonders zweckmäßig hat es sich erwiesen, wenn die offenen Kammern in der Mitte der Vorrichtung nebeneinander und zwischen zwei Küvetten angeordnet sind.

Die Vorrichtung der Erfindung ist für verschiedene Untersuchungen von Körperflüssigkeiten geeignet. Eine besonders vorteilhafte, aber nicht ausschließliche Anwendung ist dabei das Messen der Blutgerinnungszeit. Ein Verfahren und eine Vorrichtung zum Untersuchen und Messen der Blutgerinnungszeit, bei dem die Erfindung Anwendung finden kann, ist aus der EP 0 369 168 B1 bekannt, deren Inhalt hiermit als Offenbarung eingefügt wird.

Dieses Verfahren zeichnet sich dadurch aus, dass Blutplasma und Reagenz nebeneinander auf eine im wesentlichen horizontale Innenfläche einer über diese Fläche mit einer Öffnung versehenen Meßküvette eingebracht werden, dass die Meßküvette und ihr Inhalt auf die Reaktionstemperatur erwärmt werden, dass die Meßküvette in der Meßstation um im wesentlichen 90° so geschwenkt wird, dass die Innenfläche im wesentlichen senkrecht steht und Plasma und Reagenz zusammenfließen, und dass anschließend die Messung durchgeführt wird.

Blutplasma und Reagenz werden also nebeneinander auf eine im wesentlichen horizontale Fläche aufgebracht; sie haben dabei zunächst noch die Temperatur von z. B. 15°C, bei der noch keine Reaktionen auftreten. Anschließend werden dann die Meßküvette und ihr Inhalt auf die Reaktionstemperatur erwärmt, wobei aber noch keine Reaktionen zwischen Plasma und Reagenz stattfinden, da die beiden Flüssigkeiten nebeneinander angeordnet sind und sich noch nicht vermischt haben. Anschließend wird dann die Meßküvette um im wesentlichen 90° so geschwenkt, dass die Innenfläche im wesentlichen senkrecht steht, wodurch Plasma und Reagenz zusammenfließen. Anschließend kann dann die Messung durchgeführt werden.

Die Messung erfolgt dabei mit einem magnetisch anziehbaren Rührglied. Wird die Innenfläche der Küvette anfangs um einige Grad so geneigt, dass der bei der Schwenkung nach oben zu schwenkende Endbereich der Innenfläche tiefer liegt als die übrigen Bereiche der Innenfläche, so kann anfangs das Rührglied in diesem tieferen Bereich angeordnet werden. Man kann dabei zunächst die Küvette neigen und dann das Rührglied in den tieferliegenden Bereich einbringen oder aber das Rührglied erst einbringen, so dass es beim anschließenden Neigen an die gewünschte Stelle rollt. Das Rührglied fällt dann bei Beginn des Schwenkvorgangs in das Reagenz und dann in das Plasma hinein und zieht diese mit nach unten, wodurch eine bessere Durchmischung bereits am Anfang erreicht wird. Gleichzeitig sorgt das Rührglied für eine konstante Geschwindigkeit beim Transport des Reagenzes.

Bei anderen Ausführungsformen wird man die Neigung im genau umgekehrten Sinne wählen, damit das Rührglied beim Schwenken nicht durch die Flüssigkeiten hindurchfällt, was zu Spritzern und unerwünschter Verteilung der Flüssigkeiten führen könnte.

Als besonders zweckmäßig hat es sich erwiesen, wenn das Rührglied eine Metallkugel ist.

Lässt man nach der Schwenkung die Meßküvette eine begrenzte Strecke fallen und auf eine Anschlagfläche auftreffen, so werden Rührglied und Flüssigkeiten stoßartig nach unten bewegt, so dass schnell maximale Mengen der Flüssigkeiten hier vorhanden sind und durchmischt werden können.

Es ist ohne weiteres möglich, das Verfahren fließbandartig so auszugestalten, dass gleichzeitig mehrere Meßküvetten durch die einzelnen Stationen in aufeinanderfolgende Reihenfolge geführt und anschließend entsorgt werden.

Dies ist insbesondere möglich bei einer Meßküvette zum Untersuchen und Messen der Blutgerinnungszeit, die mit einer im wesentlichen horizontal angeordneten Innenfläche, einer Öffnung über dieser Innenfläche und einer ein Zusammenfließen der Flüssigkeiten behindernden Oberflächenstruktur versehen ist, die sich dadurch auszeichnet, dass sie ein magnetisch anziehbares Rührglied aufweist und dass mehrere Meßküvetten nebeneinander in einer Halterung angeordnet sind, die eine Zahnstange aufweist.

Wenn mehrere Meßküvetten in einer gemeinsamen Halterung angeordnet sind, die eine Zahnstange aufweist, so können diese mehreren Meßküvetten durch einen Zahnradantrieb durch unterschiedliche Stationen geführt werden. Auf diese Weise kann auf sehr rationelle Weise eine große Zahl von Untersuchungen in schneller Folge durchgeführt werden.

Dadurch, dass die Innenfläche im Wesentlichen horizontal anzuordnen ist, können besonders große Mengen von Plasma im Reagenz nebeneinander angeordnet werden, die aber durch die Oberflächenstrukturen daran gehindert werden, bereits in dieser Stellung der Meßküvette zusammenzufließen. Werden die Meßküvetten anschließend geschwenkt, insbesondere um ungefähr 95°, so dass sie anschließend senkrecht stehen, so können diese Oberflächenstrukturen ein Zusammenfließen nicht mehr behindern. Dies gilt insbesondere dann, wenn man die Küvette nach dem Schwenken noch auf eine Anschlagfläche herabfallen lässt, wobei hier eine Wegstrecke von 5 mm bereits ausreicht.

Die Oberflächenstrukturen können kleine wannenartige Vertiefungen für die Flüssigkeiten sein.

Zweckmäßigerweise werden durch die Oberflächenstrukturen durch eine Zwischenfläche voneinander getrennte Bereiche begrenzt. Die Flüssigkeiten verbleiben dabei zunächst in den Oberflächenbereichen und sind durch die Zwischenfläche voneinander getrennt. Die Oberflächenstrukturen können linienförmige, gratartige Vorsprünge sein, Besonders einfach sind die Oberflächenstrukturen aber herzustellen, wenn sie linienförmige Einkerbungen sind.

Wenn die Innenfläche im zunächst tieferliegenden Bereich an ihrem Rand durch zwei in der Mitte stumpfwinklig zusammenlaufende Begrenzungswände begrenzt ist, so wird ein kugelförmiges Rührglied am Anfang automatisch in die Mitte der Kante rollen, so dass es dann von dieser Mitte durch die Flüssigkeitstropfen hindurchfällt und dadurch besonders viel Flüssigkeiten mit in den Bereich mitnimmt, in dem die anschließende Messung durchgeführt werden soll. An der Fläche, auf die das Rührglied und die Flüssigkeiten treffen, ist zweckmäßigerweise eine mittige zylinderförmige Vertiefung vorgesehen, so dass hier das kugelförmige Rührglied eine kreisförmige Bewegung durchführen kann, die durch die Magnetrühreinrichtung bewirkt wird.

Zweckmäßigerweise wird vorgesehen, dass die außerhalb der zylinderförmigen Ausnehmung angeordneten Randbereiche nach innen und in der Stellung nach der Schwenkung nach unten abgeschrägt sind, wobei wenigstens teilweise diese Endbereiche eine geringere Dicke als der Durchmesser der Kugel haben. Durch diese abgeschrägten, insbesondere kugelschalenförmig abgeschrägten Bereiche wird sichergestellt, dass die Kugel auch dann schnell in die vorgesehene zylinderförmige Bahn gelangt, falls sie auf die Randbereiche auftrifft. Wenn die Endbereiche eine geringere Dicke als der Durchmesser der Kugel haben, so wird durch die Kugel nicht nur das Flüssigkeitsmaterial im zylinderförmigen Bereich durchrührt; vielmehr wird auch auf die Flüssigkeitsteile eine Rührwirkung ausgeübt, die sich in den Randbereichen, insbesondere in den Ecken einer rechteckigen Meßküvette befinden.

Verfahren und Vorrichtung haben wie bereits erwähnt den Vorteil, dass gleichzeitig sehr viele verschiedene Messungen, nämlich insgesamt bis zu 13 Bestimmungen möglich sind. Es ist leicht möglich, die entsprechenden 13 Reagenzien gekühlt zu lagern und jederzeit einen kompletten Gerinnungsstatus zu machen. Während man bei den vorbekannten Automaten drei Pumpen z. B. für die Grundbestimmungen PT (prothrombin time = Prothrombinzeit) und PTT (partial thrombin time = partielle Thrombinzeit) hatte, die lange Zeit für den Gerinnungsstatus gereicht haben, ist es jetzt auch möglich, zusätzlich TT (thrombin time = Thrombinzeit) und Fibrinogen zu messen, was bereits in vielen Krankenhäusern geschieht. Mit keinem vorbekannten Gerät kann dies pro Patient in einem Durchgang gemessen werden. Sollten sich bei diesem Gerinnungsstatus Fehler ergeben, so können und müssen zusätzlich noch die Faktoren gemessen werden. Dies summiert sich dann zu den erwähnten insgesamt 13 Bestimmungen.

Die Erfindung wird im Folgenden anhand von vorteilhaften Ausführungsformen unter Bezugnahme auf die beigefügten Zeichnungen beispielsweise beschrieben. Es zeigen:
- Fig. 1: eine erfindungsgemäße Ausführungsform in perspektivischer Darstellung;
- Fig. 2: die Ausführungsform von Fig. 1 in Draufsicht;
- Fig. 3: das Prinzip der Verwendung von Meßküvetten, die für die Erfindung verwendet werden können;
- Fig. 4: die Meßküvette im liegenden Zustand in Draufsicht;
- Fig. 5: dieselbe Meßküvette in Endansicht;
- Fig. 6: die Meßküvette in der Messstation, wobei die Meßküvette gegenüber der Darstellung von Fig. 4 um 90° um ihre Längsachse gedreht gezeigt ist; und
- Fig. 7: eine Anzahl von Meßküvetten, die an einer gemeinsamen Halterung mit einer Zahnstange zusammengefasst sind.

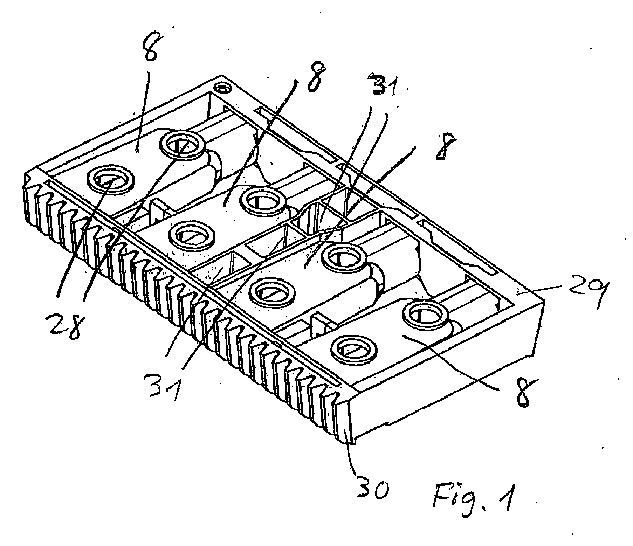

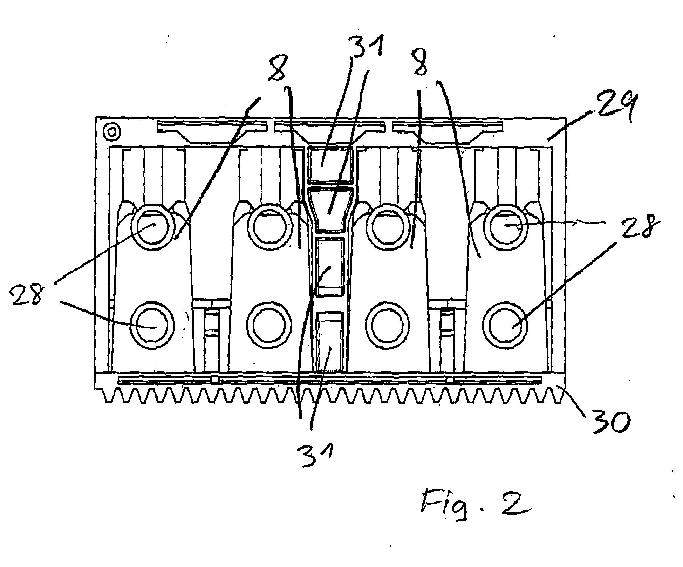

Die in den Fig. 1 und 2 gezeigte Ausführungsform der Erfindung weist mehrere Küvetten 8 auf, die mit Öffnungen 28 versehen sind, durch die Körperflüssigkeit und Reagenz eingeführt werden können. Die Küvetten 8 sind dabei in einer Halterung 29 angeordnet, die eine Zahnstange 30 aufweist, mit der die Vorrichtungen durch eine Messapparatur bewegt werden können. Zwischen den beiden mittleren Küvetten 8 weist die Halterung 29 einen Steg auf, in dem nach oben offene Kammern 31 angeordnet sind. In diese Kammern wird zunächst eine größere Menge von Körperflüssigkeit eingeführt, als für die Messung erforderlich ist. Anschließend wird dann, ohne dass Probleme wegen Druckdifferenzen auftreten können, die gewünschte Menge aus der entsprechenden Kammer 31 entnommen und in die Öffnung 28 der entsprechenden Küvette 8 eingeführt.

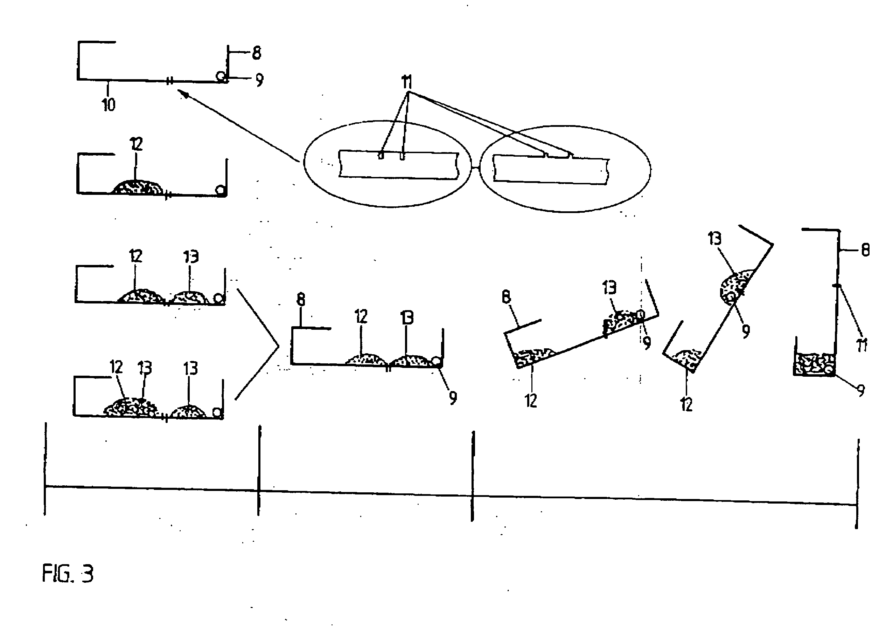

Anhand der Fig. 3 bis 7 soll nun eine besonders vorteilhafte Ausführungsform beschrieben werden. Dabei wird zunächst der in Fig. 3 gezeigte Vorgang, der mit den erfindungsgemäßen Vorrichtungen durchgeführt werden kann, erläutert.

Die Meßküvette 8 ist im Wesentlichen quaderförmig und besitzt an einer ihrer Flächen eine Öffnung, die den wesentlichen Teil dieser Flächen einnimmt. Die Meßküvette 8 hat also eine schuhähnliche Form. In Schritt 6 wird zunächst das Rührglied 9 in Form einer Kugel eingebracht. Die Meßküvette 8 ist dabei ein wenig geneigt, und zwar so, dass die Kugel 9 sich an der untersten Stellung befindet. Diese Neigung der Küvette 8 ist nicht unbedingt erforderlich und in Fig. 3 auch nicht dargestellt. In der Mitte ist die untere Fläche 10 der Küvette 8 durch eingeritzte Vertiefungen 11 oder gratartige Vorsprünge unterteilt, die im Zusammenhang mit Fig. 4 noch genauer beschrieben werden. Diese Vertiefungen bzw. Vorsprünge 11 sind in Fig. 3 auch noch vergrößert dargestellt. Links von den Einritzungen 11 wird das Plasma 12 eingebracht. Anschließend wird (in der Darstellung der Fig. 3 von oben nach unten) rechts vom Plasma 12 und den Einritzungen 11 ein Reagenz 13 eingebracht. Falls erforderlich kann dem Plasma 12 anschließend noch ein weiteres Reagenz zugeführt werden.

Die Meßküvette 8 wird im in diesem Zustand in eine weitere Station verbracht und im Schritt 7 auf eine Temperatur von 37°C inkubiert. Bei Erreichen der gewünschten Temperatur erfolgt dann im Schritt 3, d. h. in der Messstation, das Kippen der Küvette 8. Wie dies im mittleren Teil bei Schritt 3 zu sehen ist, dringt dabei die Kugel 9 in das Reagenz 13 ein und nimmt dieses mit, so dass sich in der rechten Stellung die Kugel 9 unten befindet und dabei Plasma 12 und Reagenz 13 vermischt sind. Hier erfolgt dann die Messung der Gerinnungszeit.

Die Küvette 8 ist in Fig. 4 in Draufsicht deutlicher gezeigt. Die in der Stellung von Schritt 6 und 7 von Fig. 3 untere Fläche 10, auf die Plasma 12 und Reagenz 13 aufgebracht werden, ist mit rechtwinklig zueinanderstehenden Einkerbungen versehen, die zumindestens in der Fig. 4 im oberen Bereich eine geschlossene Fläche umranden. Die beiden Bereiche, die durch die Einkerbungen 11 wenigstens teilweise umrandet sind, sind durch einen Zwischenbereich 14 getrennt, so dass die Flüssigkeiten, deren Fließen durch die Einkerbungen 11 behindert wird, deutlich voneinander getrennt sind, solange die Meßküvette 8 ihre im wesentlichen waagerechte Stellung einnimmt.

Die Seitenwände 15 und 16 sind geschlossen; ebenso die Stirnflächen 17 und 18. Ein Teil der oberen Fläche ist durch eine Abdeckung 19 verschlossen,

In der Fig. 4 oben ist die Grundfläche 10 durch Schrägflächen 20 begrenzt, so dass die Kugel 9 in der Mitte der Fläche 10 angeordnet ist, wenn die Küvette 8 in diesem Bereich etwas tiefer geneigt ist. Die gegenüberliegende Stirnfläche 18 weist eine zylindrische Ausnehmung 21 auf, wobei die Randbereiche 22 in den Ecken abgeschrägt sind, wie dies auch aus Fig. 4 ersichtlich ist. Fig. 5 stellt dabei die Stirnfläche 18 in Draufsicht dar.

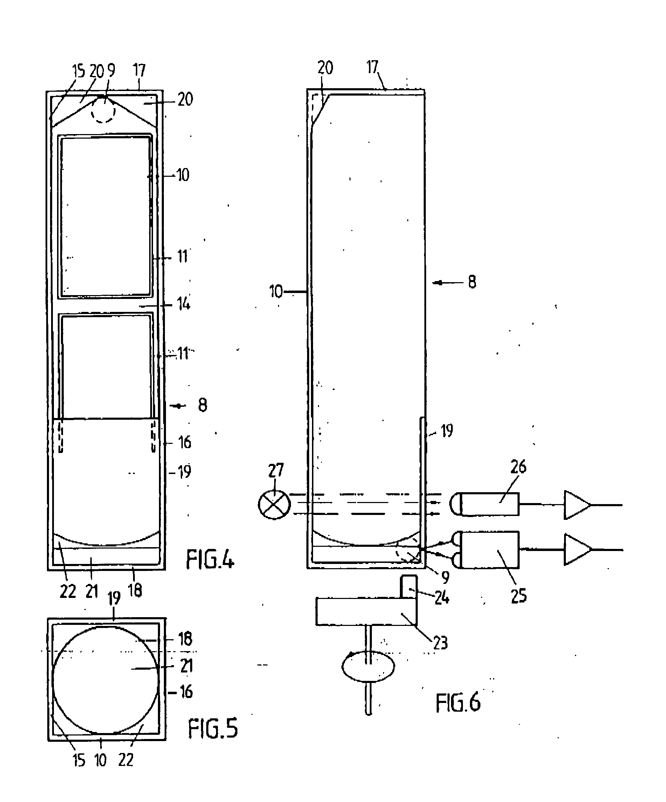

Durch die Schrägflächen 22 wird bewirkt, dass die Kugel in die zylindrische Ausnehmung 21 fällt, wenn die Meßküvette 8 in die senkrechte Stellung der Fig. 6 geschwenkt wird. Die Kugel 9 ragt dabei bis in den Raum oberhalb der zylindrischen Ausnehmung 21 hinein, so dass sie die gesamte im unteren Bereich nach dem Schwenken befindliche Flüssigkeit gut durchmischt, wenn sie durch ein Magnetrührwerk 23 mit einem Permanentmagneten 24 bewegt wird.

Der Beginn der Gerinnung kann durch photoelektrische Einrichtungen bestimmt werden, die bei 25 und 26 schematisch dargestellt sind. Die Einrichtung 25 ist dabei eine Reflektions-Messung, während die Einrichtung 26 mit einer Lichtquelle 27 eine Transmissionsmesseinrichtung ist. Diese Meßeinrichtungen sind aus den eingangs genannten Patentschriften bekannt, so dass es nicht notwendig ist, sie hier näher zu beschreiben. Es versteht sich, dass die Meßküvette durchsichtig ist, damit die Messungen durchgeführt werden können.

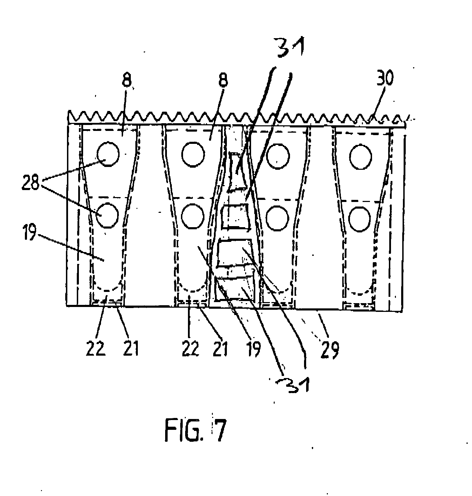

In Fig. 7 ist gezeigt, dass eine Reihe von Meßküvetten 8 nebeneinander in einer Halterung 29 angeordnet sind, die an ihrer Außenseite eine Zahnstange 30 trägt. Mit Hilfe dieser Zahnstange 30 und nicht gezeigter Zahnradantriebe kann die Halterung mit den Meßküvetten durch die einzelnen Stationen transportiert werden, so dass in schneller Folge eine große Anzahl von Untersuchungen durchgeführt werden kann. Bei der in Fig. 7 gezeigten Ausführungsform überdeckt die Abdeckplatte 19 auch mehr oder weniger den gesamten Bereich der Meßküvette 8; die Abdeckplatte 19 weist lediglich zwei Öffnungen 28 auf, durch die Plasma, Reagenz und Kugel eingegeben werden können. Bei 31 sind die erfindungsgemäßen oben offenen Kammern in der Mitte zwischen zwei Küvetten 8 gezeigt.

## Patentansprüche

1. Vorrichtung zum Untersuchen von Körperflüssigkeiten, die mehrere Küvetten (8) aufweist, in die Körperflüssigkeit und ein Reagenz einbringbar ist, **dadurch gekennzeichnet, dass** die Vorrichtung nach oben offene Kammern (31) aufweist, deren Anzahl mindestens gleichgroß ist wie diejenige der Küvetten (8).

2. Vorrichtung nach Anspruch 1, **dadurch gekennzeichnet, dass** die Anzahl der offenen Kammern (31) gleich der Anzahl der Küvetten (8) ist.

3. Vorrichtung nach Anspruch 1 oder 2, **dadurch gekennzeichnet, dass** die offenen Kammern (31) zwischen Küvetten (8) angeordnet sind.

4. Vorrichtung nach Anspruch 3, **dadurch gekennzeichnet, dass** die offenen Kammern (31) in der Mitte der Vorrichtung nebeneinander und zwischen zwei Küvetten (8) angeordnet sind.

5. Vorrichtung nach einem der Ansprüche 1 bis 4, **dadurch gekennzeichnet, dass** die Küvetten (8) eine im wesentlichen horizontal anzuordnende Innenfläche, mindestens eine Öffnung über dieser Innenfläche und ein Zusammenfließen von Körperflüssigkeit und Reagenz (12, 13) behindernde Oberflächenstrukturen (11) aufweisen und in einer Halterung (29) angeordnet sind, die die nach oben offenen Kammern (31) aufweist.

6. Vorrichtung nach Anspruch 5, **dadurch gekennzeichnet, dass** die Küvetten ein magnetisch anziehbares Rührglied aufweisen.

7. Vorrichtung nach Anspruch 5 oder 6, **dadurch gekennzeichnet, dass** die Oberflächenstrukturen (11) Vertiefungen oder kleine wannenförmige Bereiche sind.

8. Vorrichtung nach einem der Ansprüche 5 bis 7, **dadurch gekennzeichnet, dass** die Oberflächenstrukturen (11) linienförmig sind und durch diese Strukturen durch eine Zwischenfläche (14) voneinander getrennte Bereiche begrenzt sind.

9. Vorrichtung nach einem der Ansprüche 5 bis 8, **dadurch gekennzeichnet, dass** die Oberflächenstrukturen (11) linienförmige Vorsprünge sind.

10. Vorrichtung nach einem der Ansprüche 5 bis 8, **dadurch gekennzeichnet, dass** die Oberflächenstrukturen (11) linienförmige Einkerbungen sind.

11. Vorrichtung nach einem der Ansprüche 5 bis 10, **dadurch gekennzeichnet, dass** die Innenfläche (10) im zunächst tieferliegenden Bereich an ihrem Rand durch zwei in der Mitte stumpfwinklig zusammenlaufende Kanten (bei 20) begrenzt ist.

12. Vorrichtung nach Anspruch 11, **dadurch gekennzeichnet, dass** die Küvette auf der entgegengesetzten Seite in der zur Innenfläche senkrechten, nach der Schwenkung horizontalen Endfläche (18) eine mittige zylinderförmige oder ovale Vertiefung (21) aufweist.

13. Vorrichtung nach Anspruch 12, **dadurch gekennzeichnet, dass** die außerhalb der zylinderförmige in Ausnehmungen (21) angeordneten Randbereiche (22) nach innen und in der Stellung nach der Schwenkung nach unten abgeschrägt sind, wobei wenigstens teilweise diese Endbereiche (22) eine geringere Dicke als der Durchmesser des Rührglieds (Kugel 9) haben.
